# EUROPEAN PATENT APPLICATION

(11) **EP 1 918 297 A1**
(43) Date of publication of application: **07.05.2008**
(21) Application number: 06768327.6
(22) Date of filing: 20.07.2006
(51) Int. Cl.: C07K 14/00, A61K 38/00, A61P 25/00, C07K 7/06, C07K 7/08, C07K 14/435, C07K 19/00

(54) **NEURONAL DIFFERENTIATION-INDUCING PEPTIDE AND USE THEREOF**

(30) Priority: 20.07.2005 JP 2005210674; 11.08.2005 JP 2005233782; 22.09.2005 JP 2005276795
(71) Applicant: TOAGOSEI CO., LTD., Tokyo 105-8419 (JP); Kanno, Hiroshi, Yokohama-shi Kanagawa 232-20072 (JP)
(72) Inventor: YOSHIDA, Tetsuhiko, TOAGOSEI CO., LTD., Nagoya-shi, Aichi 455-0027 (JP); KOBAYASHI, Nahoko, TOAGOSEI CO., LTD., Nagoya-shi, Aichi 455-0027 (JP)
(74) Representative: Bannerman, David Gardner
(86) International application number: PCT/JP2006/314399
(87) International publication number: WO 2007/010989

(57) **Abstract**

A neuronal differentiation inducer comprising at least one peptide which can induce the differentiation of at least one cell into a neurocyte. The peptide is an artificially synthesized peptide comprising an amino acid sequence derived from a BC-box of at least one protein belonging t the SOCS protein family or an amino acid sequence having a partial modification in the BC-box-derived amino acid sequence, wherein the BC-box-derived amino acid sequence is composed of at least 10 contiguous amino acid residues selected in the amino acid sequence constituting the BC-box.

## Description

### TECHNICAL FIELD

The present invention relates to a neuronal differentiation-inducing peptide and to the use thereof. The present invention particularly relates to a neuronal differentiation inducer that has this peptide as an active ingredient.
This application claims priority from Japanese Patent Application No. 2005-210674 filed 20 July 2005, Japanese Patent Application No. 2005-233782 filed 11 August 2005, and Japanese Patent Application No. 2005-276795 filed 22 September 2005, and the contents of which are incorporated in their entirety into the present specification by reference.

### BACKGROUND ART

The regeneration of neuronal cells is a problem in the field of regenerative medicine. For example, the regeneration of neuronal cells using neural stem cells or embryonic stem cells (ES cells) has been contemplated as a treatment for various central nervous system diseases (Japanese Patent Application No. 2004-357543). However, the procurement (harvesting) of neural stem cells and the like is problematic. In addition, even when these stem cells are transplanted as such to the disease site, engraftment is also a problem as there is almost no differentiation to neuronal cells. Even in those instances where engraftment occurs, most end up differentiating into glial cells.

Somatic (adult) stem cells, such as skin stem cells and adipose stem cells can be procured relatively easily and it would be quite useful from a therapeutic perspective if these stem cells could be differentiated into neuronal cells. However, a rapid and highly efficient method for inducing the differentiation of neuronal cells from these somatic stem cells has yet to be established, and there is desire for the establishment of such a method and specifically for the development of a neuronal differentiation inducer that would satisfy this goal. For example, Japanese Patent Application No. H 9-323928 describes a neuronal differentiation inducer having a pyrrolidone derivative as its active ingredient, but does not cite an ability to induce the differentiation of neuronal cells from somatic stem cells.

### DISCLOSURE OF THE INVENTION

The present invention was created using an approach that is completely different from the conventional approach, as seen in the above-cited Japanese Patent Application No. H 9-323928, of developing neuronal differentiation inducers that employ a chemical substance as the active ingredient. An object of the present invention is to provide a peptide that has a neuronal differentiation-inducing activity. Another object is to provide a neuronal differentiation inducer (pharmaceutical composition) that has such a peptide as an active ingredient. Another object is to design such a peptide. A further object is to provide a method of producing neuronal cells using this peptide or a method of generating neuronal cells using this peptide.

The neuronal differentiation-inducing peptide provided by the present invention is an artificially designed neuronal differentiation-inducing peptide that does not exist independently in nature as a neuronal differentiation-inducing peptide. The present inventors focused on various SOCS (suppressor of cytokine signaling) proteins, which contain the SOCS box. The SOCS box is a region (amino acid sequence) capable of binding to the elongin BC complex (specifically a region of elongin C); the elongin BC complex is known to act as a transcription regulatory factor by forming a complex with elongin A. Refer, for example to
(1) PNAS, Volume 95, 1998, pp. 114-119;
(2) Genes & Development, Volume 12, 1998, pp. 3872-3881;
(3) Genes & Development, Volume 18, 2004, pp. 2867-2872; and
(4) Genes & Development, Volume 18, 2004, pp. 3055-3065 (the contents of these citations are incorporated in their entirety in this Description by reference).
   Moreover, as a result of intensive investigations, it was discovered that a high neuronal differentiation-inducing activity is exhibited on somatic stem cells by a peptide that is a portion of the peptide chains (amino acid sequence) constituting SOCS proteins and proteins in their family (collectively referred to herebelow as the "SOCS protein family") and that comprises an amino acid sequence constituting all or part of the "BC-box", a specific region that is thought to bind to the elongin BC complex. The present invention was achieved based on this discovery.

The neuronal differentiation-inducing peptide disclosed herein is a peptide that is capable of inducing the differentiation of at least one type of cell into neuronal cells (hereafter also referred to as the "neuronal differentiation-inducing peptide").
That is, as such a peptide the present invention provides an artificially synthesized peptide that comprises a BC-box-derived amino acid sequence comprising at least 10 contiguous amino acid residues selected from the amino acid sequence constituting the BC-box of any protein belonging to the SOCS protein family, or comprises an amino acid sequence obtained by partial modification of the aforesaid BC-box-derived amino acid sequence.

In a preferred embodiment of the peptide disclosed herein, an amino acid sequence constituting a membrane translocation domain (protein transduction domain)is present at the N-terminal side or C-terminal side of the BC-box-derived sequence or its modified sequence. SEQ ID NO: 16, SEQ ID NO: 17, and SEQ ID NO: 18 are preferred examples of such amino acid sequence constituting a membrane translocation domain.
In another preferred embodiment of the peptide disclosed herein, the total number of amino acid residues constituting the peptide is 50 or less.
Another preferred embodiment of the peptide disclosed herein comprises, as the BC-box-derived sequence, an amino acid sequence comprising at least 10 contiguous amino acid residues selected from the amino acid sequences shown in any of SEQ ID NO: 1 to SEQ ID NO: 15 (that is, a total of 15 sequences), or comprises an amino acid sequence obtained by partial modification of the aforesaid amino acid sequence. A preferred example is a peptide comprising an amino acid sequence shown in any of SEQ ID NO: 1 to SEQ ID NO: 15 or comprising an amino acid sequence obtained by partial modification of aforesaid sequence.

Another preferred embodiment of the peptide disclosed herein comprises, as the BC-box-derived sequence, an amino acid sequence comprising at least 10 contiguous amino acid residues selected from the amino acid sequences shown in any of SEQ ID NO: 41 to SEQ ID NO: 101 (that is, a total of 61 sequences), or comprises an amino acid sequence obtained by partial modification of the aforesaid amino acid sequence. A preferred example is a peptide comprising an amino acid sequence shown in any of SEQ ID NO: 41 to SEQ ID NO: 101 or comprising an amino acid sequence obtained by partial modification of aforesaid sequence.
Another preferred embodiment of the peptide disclosed herein comprises an amino acid sequence shown in any of SEQ ID NO: 19 to SEQ ID NO: 40, SEQ ID NO: 102, and SEQ ID NO: 103 (that is, a total of 24 sequences) or comprises an amino acid sequence obtained by partial modification of the aforesaid sequence. Peptides composed of these amino acid sequences are preferred examples.

The present invention also provides an artificially designed polynucleotide that does not exist in nature and that comprises a nucleotide sequence that encodes any of the neuronal differentiation-inducing peptides disclosed herein and/or comprises a nucleotide sequence complementary to aforesaid sequence (for example, polynucleotides substantially composed of these sequences).
A preferred polynucleotide can be exemplified by a polynucleotide comprising a nucleotide sequence that encodes an amino acid sequence as shown in any of SEQ ID NO: 1 to SEQ ID NO: 103 (that is, a total of 103 sequences) (or a modified sequence obtained by partial modification of the aforesaid amino acid sequence) and/or comprising a nucleotide sequence complementary to the aforesaid nucleotide sequence (for example, polynucleotides substantially composed of these sequences).

The present invention also provides a neuronal differentiation inducer comprising at least one of the neuronal differentiation-inducing peptides disclosed herein.
That is, the neuronal differentiation inducer disclosed herein comprises an artificially synthesized peptide that comprises a BC-box-derived amino acid sequence comprising at least 10 contiguous amino acid residues selected from the amino acid sequence constituting the BC-box of any protein belonging to the SOCS protein family, or that comprises an amino acid sequence obtained by partial modification of the aforesaid BC-box-derived amino acid sequence.

The peptide preferably comprises an amino acid sequence constituting a membrane translocation domain at the N-terminal side or C-terminal side of the BC-box-derived sequence or its modified sequence.
In addition, a preferred neuronal differentiation inducer has as an active ingredient the peptide in which the total number of amino acid residues is 50 or less.
Another preferred neuronal differentiation inducer has as an active ingredient a peptide comprising, as the BC-box-derived sequence, an amino acid sequence comprising at least 10 contiguous amino acid residues selected from an amino acid sequence shown in any of SEQ ID NO: 1 to SEQ ID NO: 15 or SEQ ID NO: 41 to SEQ ID NO: 101, or an amino acid sequence obtained by partial modification of the aforesaid amino acid sequence.
A preferred neuronal differentiation inducer has a peptide comprising an amino acid sequence shown in any of SEQ ID NO: 19 to SEQ ID NO: 40, SEQ ID NO: 102, and SEQ ID NO: 103 as an active ingredient.
The neuronal differentiation inducer disclosed herein typically comprises a pharmaceutically acceptable vehicle and one or two or more neuronal differentiation-inducing peptides.

In another aspect, the present invention provides a method of producing the neuronal differentiation-inducing peptide disclosed herein. That is, this method comprises designing a peptide chain that has the capacity to induce neuronal differentiation in at least one type of cell and that comprises a BC-box-derived amino acid sequence comprising at least 10 contiguous amino acid residues selected from the amino acid sequence constituting the BC-box of any protein belonging to the SOCS protein family, or comprises an amino acid sequence obtained by partial modification of the aforesaid BC-box-derived amino acid sequence; and synthesizing the designed peptide chain.

A peptide chain is preferably designed that comprises an amino acid sequence constituting a membrane translocation domain at the N-terminal side or C-terminal side of the BC-box-derived amino acid sequence or its modified amino acid sequence.
Moreover, the peptide chain is preferably designed in such a manner that the total number of amino acid residues constituting the peptide chain is 50 or less.

In another aspect, the present invention provides various methods for using the neuronal differentiation-inducing peptide disclosed herein.
As one of these methods, the present invention provides a method of producing neuronal cells from at least one type of cellular precursor.
That is, the method according to the present invention for producing neuronal cells comprises preparing an artificially synthesized peptide that comprises a BC-box-derived amino acid sequence comprising at least 10 contiguous amino acid residues selected from the amino acid sequence constituting the BC-box of any protein belonging to the SOCS protein family, or comprises an amino acid sequence obtained by partial modification of the aforesaid BC-box-derived amino acid sequence; and supplying the peptide to the cellular precursor.

In a preferred embodiment of this production method, the peptide comprises an amino acid sequence constituting a membrane translocation domain at the N-terminal side or C-terminal side of the BC-box-derived sequence or its modified sequence.
In another preferred embodiment, the total number of amino acid residues constituting the peptide is 50 or less.
In another preferred embodiment, the peptide comprises, as the BC-box-derived sequence, an amino acid sequence comprising at least 10 contiguous amino acid residues selected from an amino acid sequence shown in any of SEQ ID NO: 1 to SEQ ID NO: 15 or SEQ ID NO: 41 to SEQ ID NO: 101, or comprises an amino acid sequence obtained by partial modification of the aforesaid amino acid sequence.
In another preferred embodiment, the peptide comprises an amino acid sequence shown in any of SEQ ID NO: 19 to SEQ ID NO: 40, SEQ ID NO: 102, and SEQ ID NO: 103.

As a method of using the neuronal differentiation-inducing peptide disclosed herein, the present invention also provides a method of generating neuronal cells in a living organism or living tissue.
That is, the method according to the present invention for generating neuronal cells comprises preparing an artificially synthesized peptide comprising a BC-box-derived amino acid sequence that comprises at least 10 contiguous amino acid residues selected from the amino acid sequence constituting the BC-box of any protein belonging to the SOCS protein family, or comprising an amino acid sequence obtained by partial modification of the aforesaid BC-box-derived amino acid sequence; and supplying the peptide to the living organism or living tissue that has been temporarily or permanently extracted from the living organism.

In a preferred embodiment of this method of generation, the peptide comprises an amino acid sequence constituting a membrane translocation domain at the N-terminal side or C-terminal side of the BC-box-derived sequence or its modified sequence.
In another preferred embodiment, the total number of amino acid residues constituting the peptide is 50 or less.
In another preferred embodiment, the peptide comprises, as the BC-box-derived sequence, an amino acid sequence comprising at least 10 contiguous amino acid residues selected from an amino acid sequence shown in any of SEQ ID NO: 1 to SEQ ID NO: 15 or SEQ ID NO: 41 to SEQ ID NO: 101, or comprises an amino acid sequence obtained by partial modification of the aforesaid amino acid sequence.
In another preferred embodiment, the peptide comprises an amino acid sequence shown in any of SEQ ID NO: 19 to SEQ ID NO: 40, SEQ ID NO: 102, and SEQ ID NO: 103.

The peptide that is an active ingredient present in the neuronal differentiation inducer of the present invention can be easily produced since, as described above, it is a synthetic peptide comprising a BC-box-derived amino acid sequence as described above or comprising an amino acid sequence obtained by partial modification of aforesaid sequence. This enables the facile production of the peptide (and thus the neuronal differentiation inducer) in desired quantities.
In addition, the heretofore difficult induction of the differentiation of non-neuronal cells (typically somatic stem cells such as adipose stem cells, skin stem cells, and so forth, as well as embryonic stem cells) to neuronal cells is readily accomplished by using the neuronal differentiation inducer (neuronal differentiation-inducing peptide) according to the present invention as described above. As a consequence, by using a cellular precursor (e.g., adipose stem cells) that can be procured in relatively large amounts, it is then possible to supply neuronal cells in a desired amount in correspondence to the application (for example, treatment of nerve diseases that require nerve regeneration). Or, the generation of neuronal cells can be realized by the administration of a suitable quantity to a diseased site that requires nerve regeneration or to living tissue that has been temporarily or permanently extracted from a living body (including cultured material such as cell masses).

### < Sequence Listing Free Text >

SEQ ID NO: 1 to SEQ ID NO: 103 synthetic peptides

### BRIEF DESCRIPTION OF THE DRAWINGS

Figure 1 is a fluorescent photomicrograph that shows the results of an investigation, by an immune antibody procedure using the MAPs marker, of the status of neuronal differentiation after culture following the addition of sample 1 peptide at a concentration of 10 ng/mL to mouse neural stem cells.
Figure 2 is a fluorescent photomicrograph that shows the results of an investigation, by an immune antibody procedure using the NeuN marker, of the status of neuronal differentiation after culture following the addition of sample 1 peptide at a concentration of 10 ng/mL to mouse neural stem cells.
Figure 3 is a fluorescent photomicrograph that shows the results of an investigation, by an immune antibody procedure using the MAPs marker, of the status of neuronal differentiation after culture following the addition of sample 6 peptide at a concentration of 10 ng/mL to mouse neural stem cells.
Figure 4 is a fluorescent photomicrograph that shows the results of an investigation, by an immune antibody procedure using the NeuN marker, of the status of neuronal differentiation after culture following the addition of sample 6 peptide at a concentration of 10 ng/mL to mouse neural stem cells.
Figure 5 is a fluorescent photomicrograph that shows the results of an investigation, by an immune antibody procedure using the MAPs marker, of the status of neuronal differentiation after culture following the addition of sample 1 peptide at a concentration of 100 ng/mL to mouse neural stem cells.
Figure 6 is a fluorescent photomicrograph that shows the results of an investigation, by an immune antibody procedure using the NeuN marker, of the status of neuronal differentiation after culture following the addition of sample 1 peptide at a concentration of 100 ng/mL to mouse neural stem cells.
Figure 7 is a fluorescent photomicrograph that shows the results of an investigation, by an immune antibody procedure using the MAPs marker, of the status of neuronal differentiation after culture following the addition of sample 6 peptide at a concentration of 100 ng/mL to mouse neural stem cells.
Figure 8 is a fluorescent photomicrograph that shows the results of an investigation, by an immune antibody procedure using the NeuN marker, of the status of neuronal differentiation after culture following the addition of sample 6 peptide at a concentration of 100 ng/mL to mouse neural stem cells.
Figure 9 is a fluorescent photomicrograph that shows the results of an investigation, by an immune antibody procedure using the MAPs marker, of the status of neuronal differentiation after culture in the absence of peptide addition to mouse neural stem cells.
Figure 10 is a fluorescent photomicrograph that shows the results of an investigation, by an immune antibody procedure using the NeuN marker, of the status of neuronal differentiation after culture in the absence of peptide addition to mouse neural stem cells.
Figure 11 is a fluorescent photomicrograph that shows the results of an investigation, by an immune antibody procedure using the MAPs marker, of the status of neuronal differentiation after culture following the addition of sample 1 peptide at a concentration of 10 ng/mL to human adipose stem cells.
Figure 12 is a fluorescent photomicrograph that shows the results of an investigation, by an immune antibody procedure using the NeuN marker, of the status of neuronal differentiation after culture following the addition of sample 1 peptide at a concentration of 10 ng/mL to human adipose stem cells.
Figure 13 is a fluorescent photomicrograph that shows the results of an investigation, by an immune antibody procedure using the MAPs marker, of the status of neuronal differentiation after culture following the addition of sample 6 peptide at a concentration of 10 ng/mL to human adipose stem cells.
Figure 14 is a fluorescent photomicrograph that shows the results of an investigation, by an immune antibody procedure using the NeuN marker, of the status of neuronal differentiation after culture following the addition of sample 6 peptide at a concentration of 10 ng/mL to human adipose stem cells.
Figure 15 is a fluorescent photomicrograph that shows the results of an investigation, by an immune antibody procedure using the MAPs marker, of the status of neuronal differentiation after culture following the addition of sample 1 peptide at a concentration of 100 ng/mL to human adipose stem cells.
Figure 16 is a fluorescent photomicrograph that shows the results of an investigation, by an immune antibody procedure using the NeuN marker, of the status of neuronal differentiation after culture following the addition of sample 1 peptide at a concentration of 100 ng/mL to human adipose stem cells.
Figure 17 is a fluorescent photomicrograph that shows the results of an investigation, by an immune antibody procedure using the MAPs marker, of the status of neuronal differentiation after culture following the addition of sample 6 peptide at a concentration of 100 ng/mL to human adipose stem cells.
Figure 18 is a fluorescent photomicrograph that shows the results of an investigation, by an immune antibody procedure using the NeuN marker, of the status of neuronal differentiation after culture following the addition of sample 6 peptide at a concentration of 100 ng/mL to human adipose stem cells.
Figure 19 is a fluorescent photomicrograph that shows the results of an investigation, by an immune antibody procedure using the MAPs marker, of the status of neuronal differentiation after culture in the absence of peptide addition to human adipose stem cells.
Figure 20 is a fluorescent photomicrograph that shows the results of an investigation, by an immune antibody procedure using the NeuN marker, of the status of neuronal differentiation after culture in the absence of peptide addition to human adipose stem cells.

### BEST MODE FOR CARRYING OUT THE INVENTION

Suitable embodiments of the present invention are described in the following. Those matters required for execution of the present invention (for example, general matters relating to peptide synthesis, polynucleotide synthesis, and the production of a neuronal differentiation inducer containing a peptide component (pharmaceutical composition)) in addition to the items specifically described in this Description (for example, the primary structure and chain length of the neuronal differentiation-inducing peptide), can be easily grasped as matters of design variation by the individual skilled in the art based on conventional technology in the fields of medicine, pharmaceuticals, organic chemistry, biochemistry, genetic engineering, protein engineering, molecular biology, pharmacy, and so forth. The present invention can be executed based on the information disclosed in this Description and the technical common knowledge in the pertinent fields. In the text that follows, the amino acids are in some cases expressed by a single letter code according to the amino acid nomenclature given by the IUPAC-IUB Guideline (however, the three-letter code is used in the Sequence Listing).
The contents of all the documents cited in this Description are incorporated in this Description in their entirety by reference.

The "artificially synthesized neuronal differentiation-inducing peptide" cited in this Description denotes a peptide fragment whose peptide chain does not occur as such and independently in a stable manner in nature, but that can be artificially produced by chemical synthesis or biosynthesis (i.e., production based on genetic engineering) and that can exist in a stable manner in a prescribed system (for example, a composition containing the neuronal differentiation inducer).
As used in this Description, "peptide" is a term that refers to an amino acid polymer that has a plurality of peptide bonds, wherein there is no limitation on the number of amino acid residues present in the peptide chain. Thus, oligopeptides up to about 10 amino acid residues and polypeptides containing a greater number of amino acid residues are also encompassed by the neuronal differentiation-inducing peptide described in this Description.
As used in this Description, "amino acid residue" is a term that includes the N-terminal amino acid and the C-terminal amino acid of the peptide chain, unless specifically indicated otherwise.

As used in this Description, the concept of an "amino acid sequence obtained by partial modification (modified amino acid sequence)" of a prescribed amino acid sequence denotes an amino acid sequence that is generated by the substitution, deletion, and/or addition (insertion) of 1 or several amino acid residues (for example, 9 or fewer, preferably 5 or fewer, and particularly preferably 2 or 3) and that has not lost the neuronal differentiation-inducing properties possessed by the aforesaid prescribed amino acid sequence. For example, the "amino acid sequence obtained by partial modification (modified amino acid sequence)" cited in this Description encompasses sequences generated by so-called conservative amino acid replacement, in which one or several (typically 2 or 3) amino acid residues undergo a conservative substitution (for example, a sequence generated by replacing a basic amino acid residue with a different basic amino acid residue), and sequences generated by the addition (insertion) of one or several (typically 2 or 3) amino acid residues to a prescribed amino acid sequence or by the deletion of one or several (typically 2 or 3) amino acid residues from a prescribed amino acid sequence.
As used in this Description, "polynucleotide" is a term that indicates a polymer (nucleic acid) in which a plurality of nucleotides are connected by phosphodiester bonds and is not limited by the number of nucleotides. DNA fragments and RNA fragments of various lengths are encompassed by the polynucleotide of this Description. In addition, an "artificially designed polynucleotide" refers to a polynucleotide whose nucleotide chain (full length) does not occur as such in nature, but that can be artificially synthesized by chemical synthesis or biosynthesis (i.e., production based on genetic engineering).

The present inventors have identified an amino acid sequence that is an amino acid sequence present in the BC-box, which is a region (domain or motif) that binds to the elongin BC complex (specifically to a portion of the amino acid sequence moiety of elongin C), and that can manifest a neuronal differentiation-inducing activity even as an artificially synthesizable, relatively short peptide chain; the present inventors have also identified a peptide comprising aforesaid sequence.
SEQ ID NO: 1 to SEQ ID NO: 15, which are representative examples thereof, are amino acid sequences contained in the BC-box of various proteins that have been identified as SOCS family proteins (refer to the four citations given above).
Specifically, amino acid sequences, and the peptides comprising aforesaid sequences, are shown that comprise 14 or 15 contiguous amino acid residues from the N terminal of the BC-boxes present in mSOCS-1 (SEQ ID NO: 1), mSOCS-2 (SEQ ID NO: 2), mSOCS-3 (SEQ ID NO: 3), mSOCS-4 (SEQ ID NO: 4), mSOCS-5 (SEQ ID NO: 5), hSOCS-6 (SEQ ID NO: 6), hSOCS-7 (SEQ ID NO: 7), hRAR-1 (SEQ ID NO: 8), hRAR-like (SEQ ID NO: 9), mWSB-1 (SEQ ID NO: 10), mWSB-2 (SEQ ID NO: 11), mASB-1 (SEQ ID NO: 12), mASB-2 (SEQ ID NO: 13), hASB-3 (SEQ ID NO: 14), and LRR-1 (SEQ ID NO: 15) (refer to the four citations given above).

In addition, while not providing a fully detailed description, SEQ ID NO: 41 to SEQ ID NO: 101 show the amino acid sequences, and peptides comprising to aforesaid sequences, present in the BC-box of various SOCS family proteins identified in viruses (HIV, AdV, SIV, and so forth) and mammals. For example, SEQ ID NO: 96 and SEQ ID NO: 100 are the amino acid sequences present in the BC-box of a SOCS family protein (MUF1) identified in humans. SEQ ID NO: 101 is the amino acid sequence present in the BC-box of the SOCS family protein mCIS (cytokine-inducible SH2-containing protein) identified in the mouse.
These are examples, and there is no intent to limit to these examples the amino acid sequences encompassed by the present invention that constitute BC-boxes. Although examples need not be provided here, amino acid sequences constituting a variety of BC-boxes are described in quite a number of publications that had been published as of the filing date for this application. The amino acid sequences can be easily acquired by ordinary search methodologies.

A peptide comprising at least 10 contiguous amino acid residues (for example, the 10 amino acid residues on the N-terminal side of the amino acid sequences shown in the Sequence Listing) selected from the amino acid sequences labeled with these sequence identification numbers and shown in the Sequence Listing appended to this Description can be suitably used in the present invention as the neuronal differentiation-inducing peptide.
In addition, a suitable modified sequence based on an amino acid sequence (preferably an amino acid sequence comprising 10 to 15 contiguous amino acid residues) comprising at least 10 contiguous amino acid residues selected from the amino acid sequences shown in the Sequence Listing, can be used as the neuronal differentiation-inducing peptide.
For example, preferred is a peptide that exhibits neuronal differentiation inducing activity and that is an amino acid sequence generated by deleting several amino acid residues from an amino acid sequence comprising 14 or 15 contiguous amino acid residues from the N terminal of the BC-box shown in an individual sequence identification number; such an amino acid sequence can be exemplified by an amino acid sequence comprising 8, 9, 10, 11, 12, or 13 contiguous amino acid residues from the N terminal of the BC-box shown in the particular sequence identification number (that is, a modified amino acid sequence generated by deleting several amino acid residues on the C-terminal side of the BC-box shown in the particular sequence identification number).
Another very suitable modified amino acid sequence is generated by the addition of several amino acid residues on the C-terminal side of an amino acid sequence comprising 14 or 15 contiguous amino acid residues from the N terminal of the BC-box shown in a particular sequence identification number.

While the neuronal differentiation-inducing peptide may be a peptide comprising only the above-described BC-box-derived amino acid sequence or a modified amino acid sequence thereof (also collectively referred to hereafter as the "BC-box-related sequence"), the introduction of an amino acid sequence comprising a so-called membrane translocation domain (a protein transduction domain) is preferred from the standpoint of improving the neuronal differentiation-inducing activity. When supplied to a prescribed cellular precursor (a target cell), a peptide equipped with such a domain (motif) can be promptly transduced into the cell, enabling an improved neuronal differentiation inducing activity.
A number of suitable membrane translocation domains (peptide fragments) are known, with some suitable examples thereamong being shown in SEQ ID NO: 16, 17, and 18. SEQ ID NO: 16 shows an amino acid sequence, and the peptide comprising the aforesaid sequence, of the protein transduction domain present in the TAT of HIV. SEQ ID NO: 17 shows the amino acid sequence, and the peptide comprising the aforesaid sequence, of a protein transduction domain (PTD4) that is a modification of the aforesaid TAT. SEQ ID NO: 18 shows the ANT-related amino acid sequence, and the peptide comprising the aforesaid sequence, of the Antennapedia mutant of *Drosophila.*
These membrane translocation domains shown in the Sequence Listing are strictly examples, and usable domains are not limited to these. Various membrane translocation domains usable in the execution of the present invention are described in a number of publications that had been published as of the filing date for this application. The amino acid sequences of these membrane translocation domains can be easily acquired by ordinary search methodologies.

At least one amino acid residue is preferably amidated in the neuronal differentiation-inducing peptide provided by the present invention. The structural stability (for example, the resistance to proteases) of the neuronal differentiation-inducing peptide can be improved by amidating the carboxyl group of the amino acid residue (typically the C-terminal amino acid residue of the peptide chain).
The total number of amino acid residues constituting the peptide chain is desirably no more than 100 (preferably no more than 50 and particularly preferably no more than 30) in the neuronal differentiation-inducing peptide. A short peptide with such chain length can be easily synthesized chemically and the neuronal differentiation-inducing peptide can thus be easily provided. There are no particular limitations on the conformation of the peptide (spatial structure) as long as its neuronal differentiation-inducing activity is manifested in the environment of use; however, a straight-chain or helical conformation is preferred because this tends to impair the ability to function as an immunogen (antigen). It is difficult for a peptide with such conformation to make up an epitope. Viewed from these perspectives, the neuronal differentiation-inducing peptide used in the neuronal differentiation inducer preferably is a straight chain and has a relatively low molecular weight (typically 50 or less amino acid residues and particularly 30 or fewer amino acid residues).

The proportion of the BC-box-related sequence in the overall amino acid sequence (that is, the number of amino acid residues constituting the BC-box-related sequence moiety as a percentage of the total number of amino acid residues constituting the peptide chain) is not particularly limited as long as the neuronal differentiation-inducing activity is not lost, but this proportion is preferably at least 50%. All of the amino acid residues in the neuronal differentiation-inducing peptide of the present invention are preferably L-amino acids; however, all or part of the amino acid residues may be replaced with D-amino acids to the extent that the neuronal differentiation-inducing activity is not lost.

Insofar as the neuronal differentiation-inducing activity is not lost, the neuronal differentiation-inducing peptide of the present invention can in part comprise a sequence that cannot be present in the neuronal differentiation induction-related sequence. While there are no particular limitations thereon, this partial sequence is preferably a sequence that enables the maintenance of the three-dimensional shape (typically a straight-chain conformation) of the BC-box-related sequence portion in the peptide chain.

Among the neuronal differentiation-inducing peptides disclosed herein, those with a relatively short peptide chain can be easily produced based on general methods of chemical synthesis. For example, either the heretofore known solid-phase synthesis methods or liquid-phase synthesis methods may be used. A solid-phase synthesis method using Boc (t-butyloxycarbonyl) or Fmoc (9-fluorenylmethoxycarbonyl) as the amino-protecting group is suitable.
With regard to the neuronal differentiation-inducing peptide disclosed herein, a peptide chain having a desired amino acid sequence and modification (C-terminal amidation and so forth) can be synthesized by a solid-state synthesis method using a commercially available peptide synthesizer (available, for example, from PerSeptive Biosystems, Applied Biosystems, and so forth).

Alternatively, the neuronal differentiation-inducing peptide may be biosynthesized based on genetic engineering techniques. This approach is well adapted to those instances in which a polypeptide having a relatively long peptide chain is to be produced. That is, DNA is synthesized that has a nucleotide sequence (including the ATG initiation codon) that encodes the amino acid sequence of the desired neuronal differentiation-inducing peptide. A recombinant vector having a gene-expression architecture is then constructed in conformity to the host cell; this recombinant vector is constructed from the aforementioned DNA and various regulatory elements for bringing about expression of the amino acid sequence of interest within the host cell (including a promoter, ribosome binding site, terminator, enhancer, and various cis-elements that control the expression level).
Using general techniques, this recombinant vector is transferred into a prescribed host cell (for example, yeast, insect cells, plant cells, animal (mammalian) cells) and the host cell, or tissue or an organism containing the aforesaid cell, is cultured under prescribed conditions. This enables the expression and production of the target polypeptide within the cell. The polypeptide can then be isolated from the host cell (or from the medium when it has been secreted) and purified to give the target neuronal differentiation-inducing peptide.
Methods already employed in the pertinent fields can be used as such as the method for constructing the recombinant vector, the method for transferring the constructed recombinant vector into the host cell, and so forth. Since these methods themselves do not particularly characterize the present invention, their detailed description has been omitted.

For example, a fusion protein expression system can be employed in order to bring about high-efficiency mass production in a host cell. That is, a gene (DNA) encoding the amino acid sequence of the target neuronal differentiation-inducing peptide is chemically synthesized and this synthetic gene is introduced into a suitable site in an appropriate fusion protein expression vector (for example, a GST (glutathione S-transferase) fusion protein expression vector, such as the pET series available from Novagen and the pGEX series available from Amersham Biosciences). The host cell (typically *Escherichia coli)* is transformed by this vector. The obtained transformant is cultured to produce the target fusion protein. The fusion protein is then extracted and purified. The obtained purified fusion protein is thereafter digested with a prescribed enzyme (protease), and the liberated target peptide fragment (the designed neuronal differentiation-inducing peptide) is recovered by, for example, affinity chromatography. The neuronal differentiation-inducing peptide of the present invention can be produced using such heretofore known fusion protein expression systems (for example, the GST/His system available from Amersham Biosciences can be used).
Alternatively, template DNA (that is, a synthetic gene fragment containing a nucleotide sequence that encodes the amino acid sequence of the neuronal differentiation-inducing peptide) for a cell-free protein synthesis system can be constructed and the target polypeptide can be synthesized in vitro using a so-called cell-free protein synthesis system that uses the various compounds required for peptide synthesis (ATP, RNA polymerase, amino acids, and so forth). For cell-free protein synthesis systems, reference is made, for example, to the paper of Shimizu et al. (Shimizu et al., Nature Biotechnology, 19, 751-755 (2001)) and the paper of Madin et al. (Madin et al., Proc. Natl. Acad. Sci. USA, 97(2), 559-564 (2000)). At the time of the filing of this application, a number of enterprises were already carrying out the contract production of polypeptides based on the techniques described in these papers, and cell-free protein synthesis kits (for example, the PROTEIOS (trademark) wheat-germ cell-free protein synthesis kit available from Toyobo Co., Ltd. (Japan)) are commercially available.
Accordingly, once the amino acid sequence to be used (BC-box-related sequence) has been determined and the peptide chain has been designed proceeding as described above, the target neuronal differentiation-inducing peptide can be easily synthesized and produced in accordance with this amino acid sequence using a cell-free protein synthesis system. For example, the neuronal differentiation-inducing peptide of the present invention can be readily produced based on the Puresystem (registered trademark) from the Post Genome Institute Co., Ltd. (Japan).

### starting here

A single-stranded or double-stranded polynucleotide comprising a nucleotide sequence encoding the neuronal differentiation-inducing peptide disclosed herein and/or a nucleotide sequence complementary to the aforesaid sequence can be readily produced (synthesized) by heretofore known methods. That is, the nucleotide sequence corresponding to the amino acid sequence of the neuronal differentiation-inducing peptide is readily determined and made available by selecting the codons corresponding to the individual amino acid residues that constitute the designed amino acid sequence. Once the nucleotide sequence has been identified, the polynucleotide (single-stranded) corresponding to the desired nucleotide sequence can be readily obtained using, for example, a DNA synthesizer. Using the obtained single-stranded DNA as a template, a target double-stranded DNA can be obtained using various enzymatic synthesis techniques (typically PCR).
The polynucleotide provided in accordance with the present invention may take the form of DNA or RNA (e.g., mRNA). The DNA can be provided as double-stranded or single-stranded. When the DNA is provided in single-stranded form, it may be the coding strand (sense strand) or the noncoding strand (antisense strand) with the sequence complementary thereto.
The polynucleotide provided in accordance with the present invention can be used as an input for the construction of a recombinant gene (expression cassette) for the production of the neuronal differentiation-inducing peptide by various host cells or a cell-free protein synthesis system.
The present invention provides a polynucleotide comprising a nucleotide sequence coding for a neuronal differentiation-inducing peptide with a novel amino acid sequence and/or a nucleotide sequence complementary to the aforesaid nucleotide sequence. For example, an artificially designed polynucleotide is provided that comprises (or is substantially constituted of) a nucleotide sequence coding for an amino acid sequence comprising an amino acid sequence as shown in any of SEQ ID NO: 1 to SEQ ID NO: 103 or a modified sequence of the aforesaid sequence (a neuronal differentiation induction-related sequence), wherein the total number of amino acid residues constituting the peptide chain is 50 or less (and particularly preferably 30 or fewer), and/or a nucleotide sequence complementary to the aforesaid nucleotide sequence.

Suitable neuronal differentiation-inducing peptides according to the present invention have a high neuronal differentiation-inducing activity for at least one type of cell. Due to this, they are suitable for use as an active ingredient of a neuronal differentiation inducer. The neuronal differentiation-inducing peptide present in the neuronal differentiation inducer may be in salt form as long as there is no loss of neuronal differentiation-inducing activity. For example, use can be made of the acid-adduct salt of the peptide, which can be obtained by the usual methods by the addition reaction of an inorganic acid or organic acid in general use. Other salts (for example, a metal salt) are permissible as long as they have a neuronal differentiation-inducing activity.

In addition to the neuronal differentiation-inducing peptide active ingredient, the neuronal differentiation inducer can contain, in correspondence to the form of use, any of various medicinally (pharmaceutically) acceptable vehicles. The vehicles generally used in peptide drugs as diluents, excipients, and so forth, are preferred. Typical examples are water, physiological buffer solutions and various organic solvents although this can vary as appropriate in correspondence to the use and form of the neuronal differentiation inducer. Aqueous alcohol (e.g., ethanol) solutions with suitable concentrations, glycerol, and nondrying oils such as olive oil are possible. Liposomes can also be used. Secondary components that can be present in the neuronal differentiation inducer are exemplified by various fillers, extenders, binders, wetting agents, surfactants, colorants, fragrances, and so forth.
There are no particular limitations on the form of the neuronal differentiation inducer. Typical forms can be exemplified by solutions, suspensions, emulsions, aerosols, foams, granules, powders, tablets, capsules, and ointments. In addition, in order to use, for example, by injection, the neuronal differentiation inducer can be formulated as a freeze-dried material or granular material to be used for preparation of the liquid pharmaceutical by dissolution just before use in physiological saline or a suitable buffer (for example, PBS).
The process itself by which various drug formulations (compositions) are prepared using the neuronal differentiation-inducing peptide (main component) and various vehicles (secondary components) as inputs can be in accordance with heretofore methods, and a detailed description is not provided since this formulation methodology is not a characteristic feature as such of the present invention. A source of detailed information on formulation technology is, for example, Comprehensive Medicinal Chemistry, edited by Corwin Hansch, Pergamon Press (1990). The contents of this book are incorporated in their entirety in this Description by reference.

The neuronal differentiation inducer provided by the present invention can be used by a method and in a dose that correspond to the form and intended purpose of the neuronal differentiation inducer.
For example, the herein disclosed neuronal differentiation-inducing peptide containing a BC-box-related sequence (that is, a neuronal differentiation inducer containing such a peptide) can be administered in a desired amount to a patient (that is, a living organism) by intravenous, intramuscular, subcutaneous, intracutaneous, or intraperitoneal injection as the solution. Or a solid form such as a tablet can be administered per os. This enables the generation (production) of neuronal cells within the living organism from somatic stem cells that are typically present in or near the diseased area. This in turn makes it possible to effectively treat various neural diseases for which nerve regeneration is an possible treatment. For example, by a regenerative therapeutic approach, can be realized treatment of nerve diseases, such as Parkinson's disease, cerebral infarction, Alzheimer's disease, paralysis of the body due to spinal cord injury, cerebral contusion, amyotrophic lateral sclerosis, Huntington's disease, cerebral tumor, retinopathies, and so forth.

Or, neuronal cells can be very efficiently generated outside the organism (in vitro) by the administration of a suitable amount of the neuronal differentiation inducer (neuronal differentiation-inducing peptide) to a cellular precursor that has been temporarily or permanently extracted from a living body, i.e., living tissue or cell masses (for example, cultured somatic stem cells). This signifies that the desired neuronal cells can be produced in large amounts in this cellular precursor.
Moreover, similar therapeutic effect as for the direct administration of the neuronal differentiation inducer (neuronal differentiation-inducing peptide) to a living body can also be obtained by returning the neuronal cells produced in large amounts, or the cellular precursor (living tissue or cell masses) containing the aforesaid produced neuronal cells, to the living body (typically to the diseased area that requires nerve regeneration).
As is clear from the preceding description, the present invention, through the utilization of any of the neuronal differentiation-inducing peptides disclosed herein, can provide cells, cell masses or living tissues that have been induced to differentiate into neuronal cells and that are effective for the treatment of nerve diseases.

In addition, a polynucleotide that encodes the neuronal differentiation-inducing peptide of the present invention can be used as an input for use in so-called gene therapy. For example, a gene (typically a DNA segment or an RNA segment) encoding a neuronal differentiation-inducing peptide can be incorporated into a suitable vector and, by means of transduction into a desired location, can be brought about continuous expression of the neuronal differentiation-inducing peptide according to the present invention within a living organism (cell). Accordingly, polynucleotide (DNA segment, RNA segment, and so forth) encoding a neuronal differentiation-inducing peptide according to the present invention is useful as a drug for the treatment or prevention of nerve diseases, inter alia, in the patients listed above.

Several examples of the present invention are described below, but the limitation of the present invention to these examples should not be inferred.

### Example 1: Peptide synthesis

A total of 24 peptides (samples 1 to 24) were produced using the peptide synthesizer described below. The amino acid sequences of these synthetic peptides are listed in Table 1.

**Table 1**

| Sample No. | Amino acid sequence | SEQ ID NO. | Total number of residues |
|---|---|---|---|
| 1 | YARAAARQARA-SLQYLCRFVIRQYTR | 19 | 26 |
| 2 | YARAAARQARA-SLQHICRMSIRRVMS | 20 | 26 |
| 3 | YARAAARQARA-TLLSLCRVAVRRALG | 21 | 26 |
| 4 | YARAAARQARA-SLQHLCRFRIRQLVR | 22 | 26 |
| 5 | YARAAARQARA-SLKHLCRKALRSFLT | 23 | 26 |
| 6 | YARAAARQARA-TLLESSARTILHNRI | 24 | 26 |
| 7 | YARAAARQARA-PLAHLCRLRVRKAIG | 25 | 26 |
| 8 | YARAAARQARA-SLTHLCRLEIRSSIK | 26 | 26 |
| 9 | YARAAARQARA-SLQYICRAVICRCTT | 27 | 26 |
| 10 | YARAAARQARA-SLQDLCCRAVVSCTP | 28 | 26 |
| 11 | YARAAARQARA-PLQELCRQRIVAAVG | 29 | 26 |
| 12 | YARAAARQARA-TLQHFCRLAINKCT | 30 | 25 |
| 13 | YARAAARQARA-TLQHLCRKTVNGHLD | 31 | 26 |
| 14 | YARAAARQARA-SLQHICRTVICNCTT | 32 | 26 |
| 15 | YARAAARQARA-SLQYLCRFVI | 33 | 21 |
| 16 | YARAAARQARA-TLLSLCRVAV | 34 | 21 |
| 17 | YARAAARQARA-SLQHICRMSI | 35 | 21 |
| 18 | YARAAARQARA-SLQYLCRFVIRQYTK | 36 | 26 |
| 19 | YARAAARQARA-SLQYLALKALVTPKK | 37 | 26 |
| 20 | YARAAARQARA-PLMDLCRRSVRLALG | 38 | 26 |
| 21 | YARAAARQARA-SLQFLALKALISERR | 39 | 26 |
| 22 | YARAAARQARA-SLQYLCKFVI | 40 | 21 |
| 23 | YARAAARQARA-ALFELCGRAV | 102 | 21 |
| 24 | YARAAARQARA-SLQHLCRLVINRLVA | 103 | 26 |

As shown in Table 1, samples 1 to 24 all have PTD4 (SEQ ID NO: 17), which is a membrane translocation domain, on the N-terminal side and adjacently have a BC-box-related sequence on the C-terminal side. That is, samples 1, 2, 3, 4, 5, 6, 7, 8, 9, 10, 11, 12, 13, and 14 have an amino acid sequence comprising 14 or 15 contiguous amino acid residues from the N terminal of the BC-box present in, respectively, hSOCS-6 (SEQ ID NO: 6), mWSB-1 (SEQ ID NO: 10), mASB-1 (SEQ ID NO: 12), hSOCS-7 (SEQ ID NO: 7), mWSB-2 (SEQ ID NO: 11), LRR-1 (SEQ ID NO: 15), mASB-2 (SEQ ID NO: 13), hASB-3 (SEQ ID NO: 14), mSOCS-5 (SEQ ID NO: 5), hRAR-1 (SEQ ID NO: 8), mSOCS-1 (SEQ ID NO: 1), mSOCS-2 (SEQ ID NO: 2), mSOCS-3 (SEQ ID NO: 3), and mSOCS-4 (SEQ ID NO: 4).
In addition, sample 15 has an amino acid sequence comprising 10 contiguous amino acid residues from the N terminal of the BC-box present in hSOCS-6 (SEQ ID NO: 6). Sample 16 has an amino acid sequence comprising 10 contiguous amino acid residues from the N terminal of the BC-box present in mASB-1 (SEQ ID NO: 12). Sample 17 has an amino acid sequence comprising 10 contiguous amino acid residues from the N terminal of the BC-box present in mWSB-1 (SEQ ID NO: 10).
Sample 18 has a modified amino acid sequence obtained by substituting K for the C-terminal amino acid residue R in the amino acid sequence comprising 15 contiguous amino acid residues from the N terminal of the BC-box present in hSOCS-6 (SEQ ID NO: 6). Sample 19 has an amino acid sequence comprising 15 contiguous amino acid residues from the N terminal of the BC-box present in HIV-1 VIF A (SEQ ID NO: 80). Sample 20 has an amino acid sequence comprising 15 contiguous amino acid residues from the N terminal of the BC-box present in M-SSB1 (SEQ ID NO: 92). Sample 21 has an amino acid sequence comprising 15 contiguous amino acid residues from the N terminal of the BC-box present in SIV VIF CPZ-GAB (SEQ ID NO: 89). Sample 22 has a modified amino acid sequence obtained by substituting K for the R constituting the seventh amino acid residue from the N terminal in the amino acid sequence comprising 10 contiguous amino acid residues from the N terminal of the BC-box present in hSOCS-6 (SEQ ID NO: 6).
Sample 23 has an amino acid sequence comprising 10 contiguous amino acid residues from the N terminal of the BC-box present in MUF1 (SEQ ID NO: 96). Sample 24 has an amino acid sequence comprising 15 contiguous amino acid residues from the N terminal of the BC-box present in mCIS (SEQ ID NO: 101).
In addition, the carboxyl (-COOH) group of the C-terminal amino acid has been amidated (-CONH₂) in all of these samples.

Each of the peptides described above was synthesized by solid-phase synthesis (Fmoc strategy) using a commercial peptide synthesizer (model 433A from Applied Biosystems). HATU (product of Applied Biosystems) was used as the condensing agent, and the amino acids and resin used for the solid-phase synthesis were purchased from Novabiochem. Rink Amide resin (100 to 200 mesh) was used as the solid support in the amidation of the C terminal of the amino acid sequences.
Synthetic peptide with the desired chain length was obtained by extending the peptide chain from the Fmoc-amino acid bonded to the resin by repeating the deprotection reaction and condensation reaction in accordance with the synthesis program of the peptide synthesizer identified above. Specifically, the following process was repeated: cleavage and removal of Fmoc, the amino protecting group on the amino acid, with 20% piperidine/dimethylformamide (DMF) (peptide synthesis grade, from Kanto Chemical Co., Inc.); washing with DMF; reaction with 4 eq. of the particular Fmoc-amino acid (-OH); washing with DMF. After the chain-extension reaction on the peptide chain had been completed, the Fmoc group was cleaved off with 20% piperidine/DMF and the aforementioned reaction product was washed in sequence with DMF and methanol.

After the solid-phase reaction, the synthesized peptide chain was transferred in combination with the resin to a centrifuge tube; 1.8 ml ethanediol, 0.6 mL m-cresol, 3.6 mL thioanisole, and 24 mL trifluoroacetic acid were added; and stirring was carried out for 2 hours at room temperature. The resin to which the peptide chain had been bonded was thereafter removed by filtration.
Cold ethanol was then added to the filtrate and a peptide precipitate was obtained by cooling on ice-cooled water. The supernatant obtained by centrifugal separation (2500 rpm, 5 minutes) was then discarded. Cold diethyl ether was added anew; thorough stirring was carried out; and centrifugal separation was carried out under the previously mentioned conditions. This stirring/centrifugal separation treatment was carried out repetitively for a total of 3 times.

The obtained peptide precipitate was dried under vacuum and was purified using a high-performance liquid chromatograph (Waters 600 from Waters Corporation).
In specific terms, a pre-column (Guard-Pak Delta-pak C18 A300 from Nihon Waters K.K.) and a C18 reverse-phase column (Nihon Waters K.K., XTerra (registered trademark) column, MS C18, 5 µm, 4.6 × 150 mm) were used, and a mixture of 0.1% aqueous trifluoroacetic acid solution and a solution of 0.1% trifluoroacetic acid in acetonitrile was used as the elutant. That is, separation/purification was carried out over 30 to 40 minutes on the previously cited column at a flow rate of 1.5 mL/minute while carrying out a timewise increase in the proportion of the trifluoroacetic acid/acetonitrile solution in the elutant (a concentration gradient from 10% to 80% as the volumetric ratio was set up). The peptide eluting from the reverse-phase column was detected at a wavelength of 220 nm using an ultraviolet detector (490E Detector, Waters Corporation) and appeared as a peak on the recoding chart.
The molecular weight of each eluted peptide was determined based on matrix-assisted laser desorption time-of-flight mass spectrometry (MALDI-TOF/MS) using a Voyager DE RP (trade name) from PerSeptive Biosystems. The results confirmed the synthesis and purification of the target peptide.

### Example 2: Evaluation of the neuronal differentiation-inducing activity of the synthetic peptides

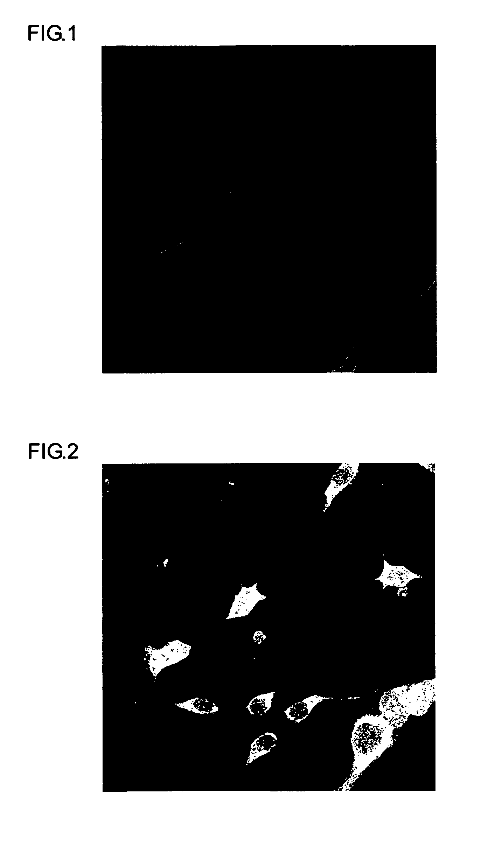

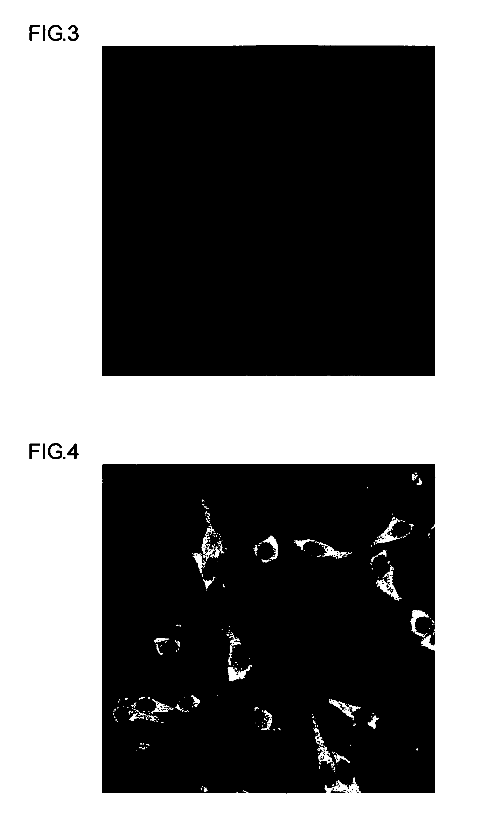

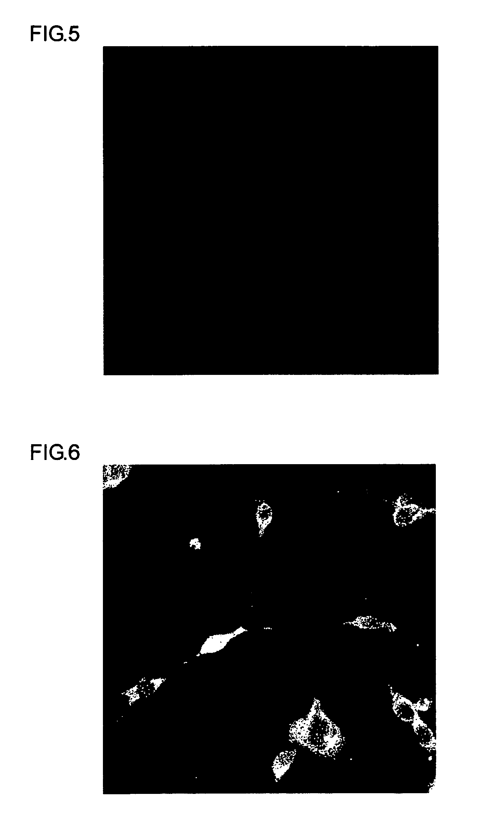

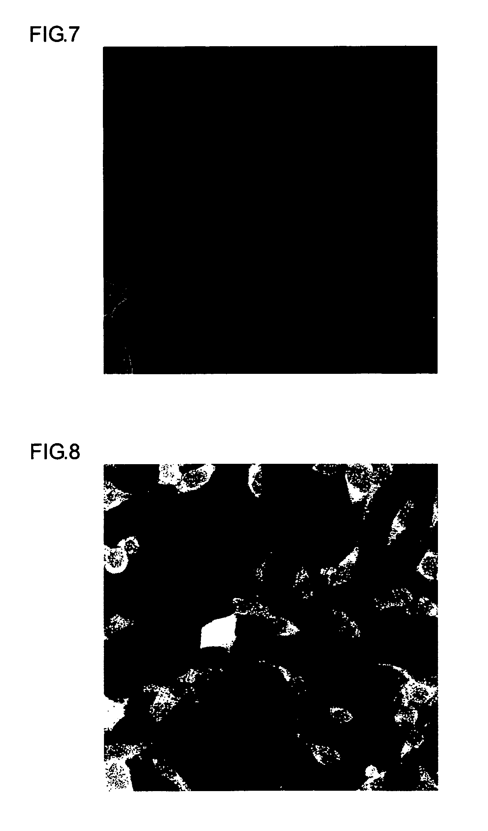

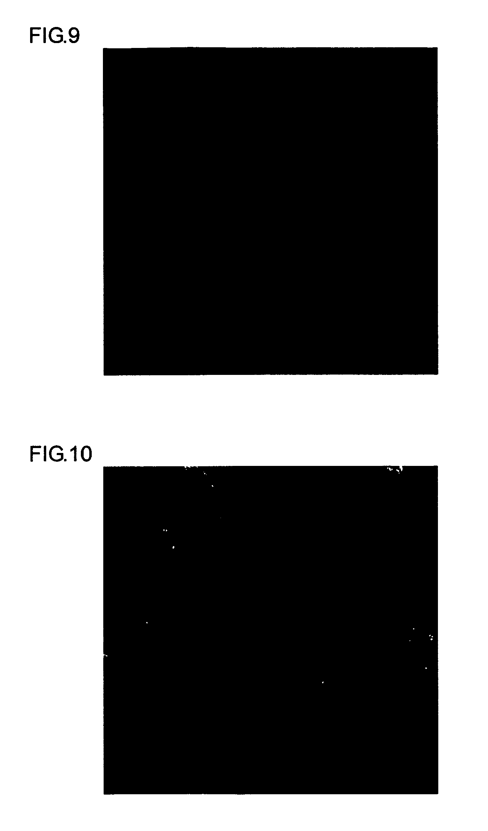

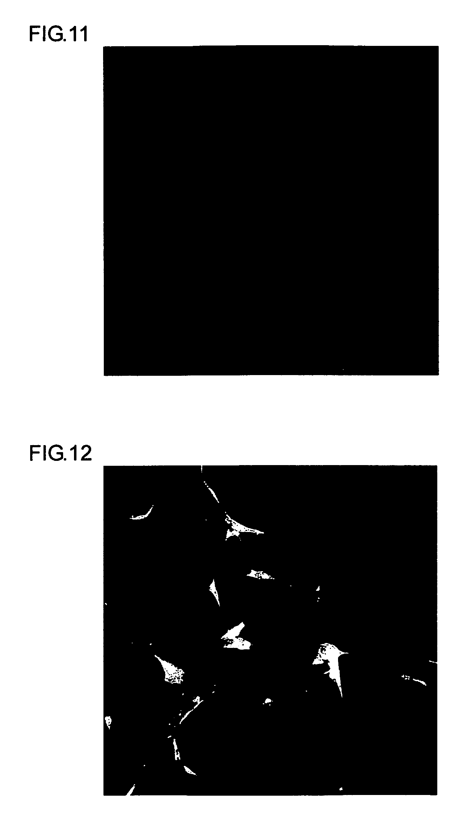

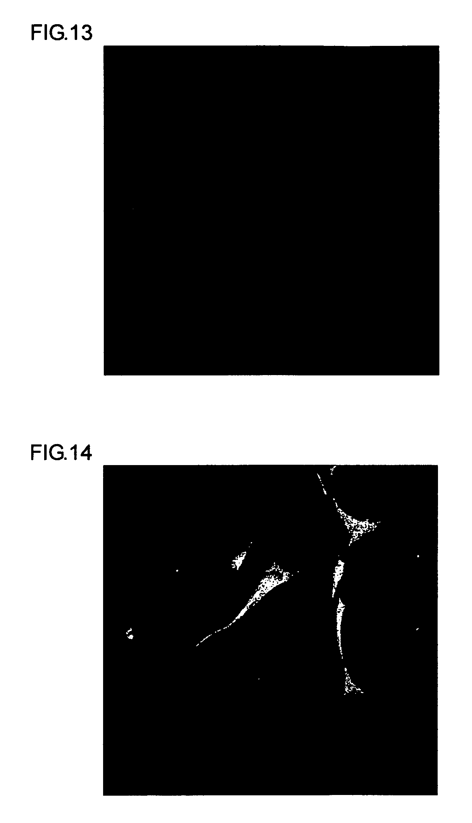

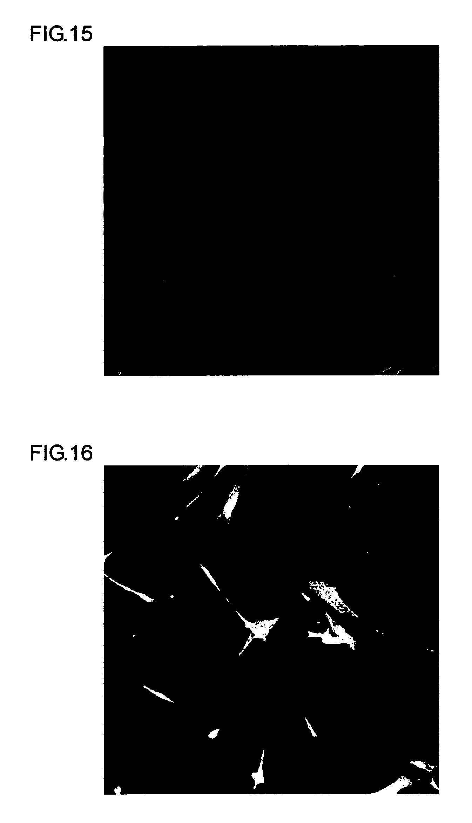

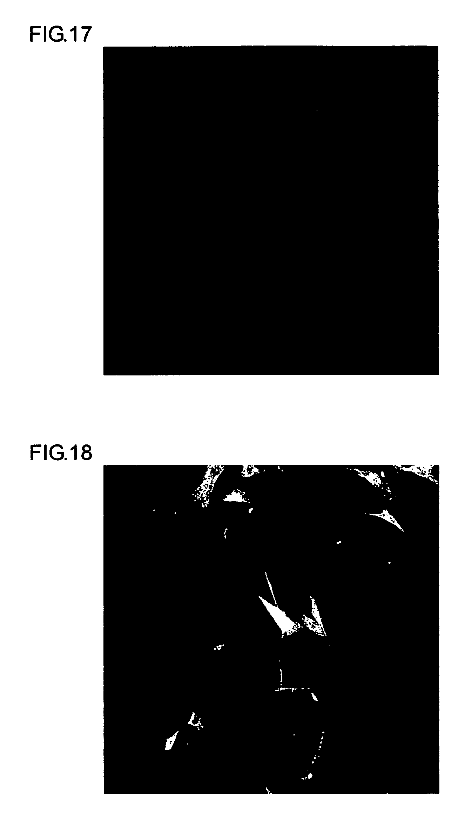

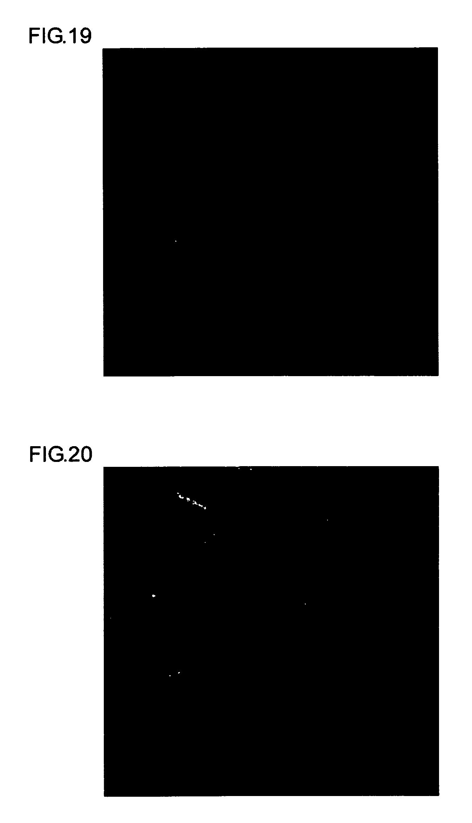

Among the neuronal differentiation-inducing peptides obtained in Example 1, the neuronal differentiation-inducing activity was investigated for the two peptides of sample 1 (YARAAARQARA-SLQYLCRFVIRQYTR) and sample 6 (YARAAARQARA-TLLESSARTILHNRI).
That is, these sample peptides were added to and incubated in a culture of neural stem cells procured from mice and a culture of adipose stem cells procured from humans. Addition concentrations of 10 ng/mL and 100 ng/mL were used for both peptides.
At 24 hours after peptide addition, evaluation was carried out by a standard immune antibody procedure. Specifically, MAPs (microfilament associated proteins) and NeuN (neuronal nuclei) were added as neuron markers, and the degree of neuronal differentiation was evaluated by confocal laser microscopy. MAPs is a marker that can stain (detect) broadly, from relatively immature neurons to mature neurons. NeuN is a marker that can specifically stain (detect) mature neurons.
The results, as shown in the photomicrographs in Figures 1 to 20, confirmed significant neuronal differentiation for both sample peptides (at 10 ng/mL concentration) for the both types of stem cells as cited above. An even more significant neuronal differentiation was observed at a concentration of 100 ng/mL. Such neuronal differentiation was not observed in the control, in which incubation was carried out without the addition of the sample peptide.
The same tests as described above were carried out on sample 2 (YARAAARQARA-SLQHICRMSIRRVMS), sample 3 (YARAAARQARA-TLLSLCRVAVRRALG), sample 4 (YARAAARQARA-SLQHLCRFRIRQLVR), sample 5 (YARAAARQARA-SLKHLCRKALRSFLT), sample 7 (YARAAARQARA-PLAHLCRLRVRKAIG), sample 8 (YARAAARQARA-SLTHLCRLEIRSSIK), sample 9 (YARAAARQARA-SLQYICRAVICRCTT), sample 10 (YARAAARQARA-SLQDLCCRAVVSCTP), sample 11 (YARAAARQARA-PLQELCRQRIVAAVG), sample 12 (YARAAARQARA-TLQHFCRLAINKCT), sample 13 (YARAAARQARA-TLQHLCRKTVNGHLD), sample 14 (YARAAARQARA-SLQHICRTVICNCTT), sample 15 (YARAAARQARA-SLQYLCRFVI), sample 16 (YARAAARQARA-TLLSLCRVAV), sample 17 (YARAAARQARA-SLQHICRMSI), sample 18 (YARAAARQARA-SLQYLCRFVIRQYTK), sample 19 (YARAAARQARA-SLQYLALKALVTPKK), sample 20 (YARAAARQARA-PLMDLCRRSVRLALG), sample 21 (YARAAARQARA-SLQFLALKALISERR), sample 22 (YARAAARQARA-SLQYLCKFVI), sample 23 (YARAAARQARA-ALFELCGRAV), and sample 24 (YARAAARQARA-SLQHLCRLVINRLVA), and significant neuronal differentiation was also confirmed for all of these samples for the both types of stem cells as cited above.
The preceding confirmed that neuronal differentiation-inducing peptides (neuronal differentiation inducers) according to the present invention could bring about the effective differentiation of cells, e.g., stem cells, into neuronal cells (neurons).

### Example 3: Granule production

50 mg of the sample 1 peptide, 50 mg microcrystalline cellulose, and 400 mg lactose were mixed; 1 mL of a mixed solution of ethanol and water was added; and mixing/kneading was carried out. The resulting mixture was granulated by a standard method to obtain granules in which the neuronal differentiation-inducing peptide was the principal component (granular neuronal differentiation inducer).

While specific examples of the present invention have been described in detail in the preceding, these are nothing more than examples and do not limit the scope of the claims. The various modifications and alterations of the specific examples provided as examples above are encompassed in the technology described in the claims.

### INDUSTRIAL APPLICABILITY

The neuronal differentiation-inducing peptide of the present invention as described hereinabove can be utilized as a peptide component in drugs and pharmaceuticals because it exhibits a high neuronal differentiation-inducing activity.

## Claims

1. A neuronal differentiation inducer comprising at least one peptide that is capable of inducing the differentiation of at least one type of cell into a neuronal cell, wherein the neuronal differentiation inducer comprises, as the peptide, an artificially synthesized peptide that comprises a BC-box-derived amino acid sequence comprising at least 10 contiguous amino acid residues selected from the amino acid sequence constituting the BC-box of any protein belonging to the SOCS protein family, or that comprises an amino acid sequence obtained by partial modification of the aforesaid BC-box-derived amino acid sequence.

2. The neuronal differentiation inducer according to claim 1, wherein the peptide comprises an amino acid sequence constituting a membrane translocation domain at the N-terminal side or C-terminal side of the BC-box-derived sequence or its modified sequence.

3. The neuronal differentiation inducer according to claim 1 or 2, wherein the total number of amino acid residues constituting the peptide is 50 or less.

4. The neuronal differentiation inducer according to claim 3, wherein the peptide comprises, as the BC-box-derived sequence, an amino acid sequence comprising at least 10 contiguous amino acid residues selected from an amino acid sequence shown in any of SEQ ID NO: 1 to SEQ ID NO: 15 and SEQ ID NO: 41 to SEQ ID NO: 101, or comprises an amino acid sequence obtained by partial modification of the aforesaid amino acid sequence.

5. The neuronal differentiation inducer according to claim 3, wherein the peptide comprises an amino acid sequence shown in any of SEQ ID NO: 19 to SEQ ID NO: 40, SEQ ID NO: 102, and SEQ ID NO: 103.

6. A synthetic peptide that is at least one artificially synthesized peptide capable of inducing the differentiation of at least one type of cell into a neuronal cell, the synthetic peptide comprising:
a BC-box-derived amino acid sequence comprising at least 10 contiguous amino acid residues selected from the amino acid sequence constituting the BC-box of any protein belonging to the SOCS protein family, or an amino acid sequence obtained by partial modification of the aforesaid BC-box-derived amino acid sequence; and
an amino acid sequence constituting a membrane translocation domain at the N-terminal side or C-terminal side of the BC-box-derived amino acid sequence or its modified amino acid sequence.

7. The synthetic peptide according to claim 6, wherein the total number of amino acid residues constituting the peptide is 50 or less.

8. The synthetic peptide according to claim 7, wherein the peptide comprises, as the BC-box-derived sequence, an amino acid sequence comprising at least 10 contiguous amino acid residues selected from an amino acid sequence shown in any of SEQ ID NO: 1 to SEQ ID NO: 15 and SEQ ID NO: 41 to SEQ ID NO: 101, or comprises an amino acid sequence obtained by partial modification of the aforesaid amino acid sequence.

9. The synthetic peptide according to claim 7, comprising an amino acid sequence shown in any of SEQ ID NO: 19 to SEQ ID NO: 40, SEQ ID NO: 102, and SEQ ID NO: 103.

10. A method of producing a peptide that is able to induce the differentiation of at least one type of cell into a neuronal cell, the method comprising:
designing a peptide chain that has the capacity to induce neuronal differentiation in at least one type of cell and that comprises a BC-box-derived amino acid sequence comprising at least 10 contiguous amino acid residues selected from the amino acid sequence constituting the BC-box of any protein belonging to the SOCS protein family, or comprises an amino acid sequence obtained by partial modification of the aforesaid BC-box-derived amino acid sequence; and
synthesizing the designed peptide chain.

11. The method according to claim 10, wherein a peptide chain is designed that comprises an amino acid sequence constituting a membrane translocation domain at the N-terminal side or C-terminal side of the BC-box-derived sequence or its modified amino acid sequence.

12. The method according to claim 10 or 11, wherein the peptide chain is designed in such a manner that the total number of amino acid residues constituting the peptide chain is 50 or less.

13. A method of producing a neuronal cell from at least one type of cellular precursor, comprising:
providing an artificially synthesized peptide that comprises a BC-box-derived amino acid sequence comprising at least 10 contiguous amino acid residues selected from the amino acid sequence constituting the BC-box of any protein belonging to the SOCS protein family, or comprises an amino acid sequence obtained by partial modification of the aforesaid BC-box-derived amino acid sequence; and
supplying the peptide to the cellular precursor.

14. The method according to claim 13, wherein the peptide comprises an amino acid sequence constituting a membrane translocation domain at the N-terminal side or C-terminal side of the BC-box-derived sequence or its modified sequence.

15. The method according to claim 13 or 14, wherein the total number of amino acid residues constituting the peptide is 50 or less.

16. The method according to claim 15, wherein the peptide comprises, as the BC-box-derived sequence, an amino acid sequence comprising at least 10 contiguous amino acid residues selected from an amino acid sequence shown in any of SEQ ID NO: 1 to SEQ ID NO: 15 and SEQ ID NO: 41 to SEQ ID NO: 101, or comprises an amino acid sequence obtained by partial modification of the aforesaid amino acid sequence.

17. The method according to claim 15, wherein the peptide comprises an amino acid sequence shown in any of SEQ ID NO: 19 to SEQ ID NO: 40, SEQ ID NO: 102, and SEQ ID NO: 103.

18. A method of generating neuronal cells in a living organism or in living tissue, the method comprising:
providing an artificially synthesized peptide that comprises a BC-box-derived amino acid sequence comprising at least 10 contiguous amino acid residues selected from the amino acid sequence constituting the BC-box of any protein belonging to the SOCS protein family, or comprises an amino acid sequence obtained by partial modification of the aforesaid BC-box-derived amino acid sequence; and
supplying the peptide to the living organism or to living tissue that has been temporarily or permanently extracted from the living organism.

19. The method according to claim 18, wherein the peptide comprises an amino acid sequence constituting a membrane translocation domain at the N-terminal side or C-terminal side of the BC-box-derived sequence or its modified sequence.

20. The method according to claim 18 or 19, wherein the total number of amino acid residues constituting the peptide is 50 or less.

21. The method according to claim 20, wherein the peptide comprises, as the BC-box-derived sequence, an amino acid sequence comprising at least 10 contiguous amino acid residues selected from an amino acid sequence shown in any of SEQ ID NO: 1 to SEQ ID NO: 15 and SEQ ID NO: 41 to SEQ ID NO: 101, or comprises an amino acid sequence obtained by partial modification of the aforesaid amino acid sequence.

22. The method according to claim 20, wherein the peptide comprises an amino acid sequence shown in any of SEQ ID NO: 19 to SEQ ID NO: 40, SEQ ID NO: 102, and SEQ ID NO: 103.
